# EUROPÄISCHE PATENTSCHRIFT

(11) **EP 0 646 564 B1**
(45) Veröffentlichungstag und Bekanntmachung des Hinweises auf die Patenterteilung: **18.06.1997**
(21) Anmeldenummer: 94115087.2
(22) Anmeldetag: 24.09.1994
(51) Int. Cl.: C07C 29/80, C08G 63/78

(54) **Verfahren zur Aufarbeitung von Butandiol**
Method of working up butanediol
Procédé de traitement de butanediol

(30) Priorität: 05.10.1993 DE 4333929
(43) Veröffentlichungstag der Anmeldung: 05.04.1995
(73) Patentinhaber: BASF Aktiengesellschaft, 67063 Ludwigshafen (DE)
(72) Erfinder: Braune, Peter, Dr., D-55234 Erbes-Büdesheim (DE)

(56) Entgegenhaltungen:
- FR-A- 2 306 228
- US-A- 4 239 882
- US-A- 4 499 261

## Beschreibung

Die vorliegende Erfindung betrifft ein Verfahren zur Aufarbeitung von Dihydroxyverbindungen enthaltenden Rückständen wie sie bei der Herstellung von Polyestern durch Umsetzung von Dicarbonsäuren bzw. deren Estern oder esterbildenden Derivaten mit Dihydroxyverbindungen anfallen, wobei
a) in einer ersten Stufe ein Diester einer Dicarbonsäure mit einem Alkanol mit einem molaren Überschuß einer Dihydroxyverbindung umgeestert wird,
b) die bei der Umsetzung gemäß a) anfallenden Brüden, die überwiegend das bei der Umesterung gebildete Alkanol, überschüssige Dihydroxyverbindungen sowie oligomere und polymere Reaktionsprodukte enthalten, in eine Kolonne überführt werden, in der das Alkanol über Kopf und die anderen Produkte als Sumpfprodukt abgetrennt werden, und
c) das Sumpfprodukt anschließend einer weiteren Behandlung zur Rückgewinnung der Dihydroxyverbindung unterworfen wird.

Zur Herstellung von Polyestern, insbesondere Polyalkylenterephthalaten werden in großem Umfang sogenannte Umesterungs/Polykondensationsverfahren ausgeführt, wobei in einer ersten Stufe eine Umesterung und in mindestens einer weiteren Stufe die eigentliche Polykondensation vorgenommen wird (vgl. Chemiefasern/Textilindustrie 40 (1992), 1058-1062 und Ullmann's Enzyklopädie der technischen Chemie, 4. Aufl. Band 19, S.61-88).

Am Beispiel der Herstellung von Polybutylenterephthalat aus Dimethylterephthalat und Butandiol-1,4 sei dieses Verfahren kurz erläutert.

In einem ersten Reaktionsraum wird Dimethylterephthalat mit einem molaren Überschuß, vorzugsweise 5-60 mol%, insbesondere 10-45 mol%, Butandiol-1,4 umgeestert, wobei die umgeesterte Verbindung in weiteren Schritten der eigentlichen Polykondensation unterworfen wird. Die bei der Umesterung anfallenden Brüden werden in eine Kolonne überführt, in der das leichtsiedende Methanol über Kopf und ein Sumpfprodukt, welches neben überschüssigem Butandiol-1,4 noch geringe Mengen Oligomere, Polymere und Dimethylterephthalat enthält, erhalten werden.

In diese Kolonne können des weiteren die bei der eigentlichen Polykondensation anfallenden Bestandteile, die hauptsächlich aus Butandiol-1,4, Oligomeren, Polymeren und Dimethylterephthalat bestehen, geleitet werden.

Außerdem können in den Sumpf dieser Kolonne zur Entfernung des nicht umgesetzten Dimethylterephthalats 0,05 bis 0,1 kg eines Ver- oder Umesterungskatalysators, beispielsweise Tetrabutylorthotitanat, pro 250 kg Sumpfaustrag eingesetzt werden.

Aus wirtschaftlichen Gründen ist es angezeigt, dieses Sumpfprodukt einer weiteren Behandlung zu unterwerfen, um das Butandiol-1,4, welches in erheblichen Anteilen enthalten ist, zurückzugewinnen. Da das bei den bekannten Verfahren anfallende Sumpfprodukt jedoch aufgrund seiner hohen Viskosität bzw. dem Charakter als Feststoff (abhängig von der Zusammensetzung) praktisch nicht mehr kontinuierlich aus der Kolonne zu entfernen ist, da es nicht mehr förderbar ist, treten häufig Störungen in dieser sogenannten "Methanolkolonne" auf, die das Verfahren beeinträchtigen. Darüber hinaus fallen bei der Aufarbeitung dieser Rückstände wiederum Feststoffe als Sumpfprodukt an, deren Verbrennung wiederum fördertechnisch aufwendig und teuer ist.

Eine abschnittsweise Spülung der Kolonnen, um die anfallenden Rückstände auszuwaschen, ist einem kontinuierlichen Verfahren ebenfalls abträglich.

Der vorliegenden Erfindung lag daher die Aufgabe zugrunde, die Aufarbeitung der Brüden in der Methanolkolonnen bei den vorstehend beschriebenen Verfahren (die, darauf sei hier nochmals hingewiesen, prinzipiell für die Herstellung von Polyestern allgemein und nicht nur für die Herstellung von Polybutylenterephthalat angewandt werden können), dahingehend zu verbessern, daß die anfallenden Sumpfprodukte leichter aus der Kolonne entfernt und zur Wiedergewinnung der Dihydroxyverbindungen gefördert werden können.

Überraschenderweise wurde gefunden, daß diese Aufgabe dadurch gelöst werden kann, daß in Stufe b) eines Verfahrens gemäß Oberbegriff des Anspruchs ein flüssiger, Dihydroxyverbindungen enthaltender Rückstand zugegeben und das Sumpfprodukt in weitgehend flüssiger Form aus der Kolonne ausgetragen und anschließend einer Behandlung zur Rückgewinnung der Dihydroxyverbindung unterworfen wird.

Durch die Zugabe dieses Dihydroxyverbindungen enthaltenden Rückstandes bleibt das Sumpfprodukt in der Kolonne in Stufe b) des Verfahrens flüssig bzw. förderbar und kann daher auf verfahrenstechnisch einfache Weise in eine weiter Kolonne überführt werden, in der die Dihydroxyverbindung über Kopf und ein wiederum flüssiges bzw. förderbares Sumpfprodukt am Boden dieser Kolonne erhalten werden, welches ebenfalls einfach der Verbrennung zugeführt werden kann.

Insgesamt ergibt sich daher eine wesentliche Vereinfachung der Behandlung der Dihydroxyverbindungen enthaltenden Rückstände, was erhebliche Einsparungen mit sich bringt.

Nachstehend sei das erfindungsgemäße Verfahren wiederum am Beispiel der Herstellung von Polybutylenterephthalat erläutert; es sei jedoch nochmals betont, daß es entsprechend auch für die Herstellung anderer dem Fachmann bekannter Polyester geeignet ist.

Zunächst werden Dimethylterephthalat und Butandiol-1,4 (letzteres in 5-60 mol%, vorzugsweise 10-45 mol% Überschuß) in an sich bekannter Weise bei Temperaturen im Bereich von 150 bis 220°C und Drücken im Bereich von 0,7 bis 1,5 bar für einen Zeitraum von 30 bis 90, vorzugsweise von 40 bis 70 Minuten miteinander umgesetzt, wobei eine Umesterung stattfindet und entstehendes Methanol zusammen mit überschüssigem Butandiol und geringen Mengen oligomerer und polymerer Verbindungen sowie Restmengen Dimethylterephthalat mit den Brüden in eine Kolonne überführt werden, in die parallel ein flüssiger Dihydroxyverbindungen enthaltender Rückstand, wie er beispielsweise bei der Destillation von Butandiol-1,4 oder Hexandiol-1,6 anfällt, eingeführt wird. Die Zusammensetzung des Rückstands unterliegt an sich keiner besonderen Beschränkung, solange die flüssige Form gegeben und keine die Trennung in der Kolonne störenden Verbindungen enthalten sind. Dies ist bei den geschilderten Rückständen aus der Butandiol bzw. Hexandiol-Destillation in aller Regel der Fall.

Der Ort der Zugabe liegt vorzugsweise in der Mitte oder im unteren Teil der Kolonne und die Zugabemenge beträgt im allgemeinen 0,05 bis 5 kg pro kg in die Kolonne überführten Brüden, vorzugsweise von 0,1 bis 0,3 kg/kg.

In der Kolonne in Stufe b) des erfindungsgemäßen Verfahrens wird das leichtsiedende Methanol über Kopf abgetrennt und der zugegebene Dihydroxyverbindungen enthaltende Rückstand zusammen mit dem üblichen Sumpfprodukt aus der Kolonne ausgetragen. Da das auszutragende Produkt infolge der Zugabe des beschriebenen Rückstandes flüssig oder zumindest förderbar ist, ist der Austrag verfahrenstechnisch auf einfache Weise und insbesondere kontinuierlich möglich, was bei einer wachsartigen bzw. pastösen oder festen Konsistenz der Sumpfprodukte, wie sie nach den bislang bekannten Verfahren erhalten wurden, nicht möglich war.

Diese erhebliche Vereinfachung des Handlings dieses Rückstands bringt berrächtliche Kosteneinsparungen mit sich.

Ein weiterer entscheidender Vorteil des erfindungsgemäßen Verfahrens liegt darin, daß der bei der Aufarbeitung des aus der Kolonne in Stufe b) anfallenden Sumpfprodukts mit dem Ziel der Wiedergewinnung von Butandiol ebenfalls flüssig bzw. förderbar bleibt und somit wiederum verfahrenstechnisch auf einfache Weise der Verbrennung zugeführt werden kann. Dies bringt gegenüber den bislang bekannten Verfahren nochmals eine Vereinfachung und Kostenersparnis mit sich.

Somit ergibt sich insgesamt, daß durch die Zugabe eines Dihydroxyverbindungen enthaltenden Rückstands in Stufe b) eines Umesterungs/Polykondensationsverfahrens erhebliche Kostenvorteile erzielt werden, ohne beispielsweise die Menge der letztlich zu verbrennenden Stoffe signifikant zu erhöhen. Da die erfindungsgemäß zugegebenen Rückstände ohnehin der Verbrennung zugeführt werden würden, was letztlich nach dem erfindungsgemäßen Verfahren ebenfalls vorteilhaft erfolgt, fallen auch keine zusätzlichen Kosten an, die den Kostenvorteil schmälern könnten.

Prinzipiell wird der zugegebene Rückstand lediglich in der Kolonne in Stufe b) und der nachfolgenden Aufarbeitungskolonne als Transportmedium für die ansonsten nicht mehr förderbaren Sumpfprodukte verwendet, ohne selbst in die Trennvorgänge oder Reaktionen einzugreifen.

### Beispiel

In einem Rührkessel wurden 992 kg/h Dimethylterephthalat, 640 kg/h Butandiol-1,4 und 1 kg/h Tetrabutylorthotitanat als Katalysator bei einer Temperatur von 195°C und einem Druck von 1 bar und einer mittleren Verweilzeit von 45 Minuten umgesetzt. Die bei der Umsetzung freiwerdenden Brüden wurden kontinuierlich in eine Kolonne überführt, in deren Mitte 80 kg/h eines bei der Butandiol-1,4-Destillation angefallenen flüssigen Rückstands (enthielt als Hauptbestandteile Butandiol-1,4, 2-Methylpentandiol-1,5, Hexandiol-1,6, 2-Methylhexandiol-1,6, Petantriol-1,2,5) zugegeben wurden.

Methanol wurde am Kopf dieser Kolonne und ein flüssiges Sumpfprodukt am Boden der Kolonne kontinuierlich ausgetragen. Das Sumpfprodukt wurde in einer weiteren Kolonne in Butandiol und ein förderbares Sumpfprodukt aufgetrennt, und letzteres über eine Rohrleitung einer Verbrennungseinrichtung zugeführt.

Die Kolonne zur Abtrennung des Methanols konnte störungsfrei betrieben und der Rückstand störungsfrei über eine Rohrleitung ausgetragen werden; ohne eine Zugabe des Rückstandes wie nach dem erfindungsgemäßen Verfahren wies das Sumpfprodukt eine wachsartige bis pastöse Konsistent auf und konnte nicht störungsfrei über Rohrleitungen ausgetragen werden.

## Patentansprüche

1. Verfahren zur Aufarbeitung von Dihydroxyverbindungen enthaltenden Rückständen, die bei der Herstellung von Polyestern durch Umsetzung von Dicarbonsäuren bzw. deren Estern oder esterbildenden Derivaten mit Dihydroxyverbindungen anfallen, wobei
a) in einer ersten Stufe ein Diester einer Dicarbonsäure mit einem Alkanol mit einem molaren Überschuß einer Dihydroxyverbindung umgeestert wird,
b) die bei der Umsetzung gemäß a) anfallenden Brüden, die überwiegend das bei der Umesterung gebildete Alkanol, überschüssige Dihydroxyverbindungen sowie oligomere und polymere Reaktionsprodukte enthalten, in eine Kolonne überführt werden, in der das Alkanol über Kopf und die anderen Produkte als Sumpfprodukt abgetrennt werden, und
c) das Sumpfprodukt anschließend einer weiteren Behandlung zur Rückgewinnung der Dihydroxyverbindung unterworfen wird,
dadurch gekennzeichnet, daß in Stufe b) des Verfahrens ein flüssiger, Dihydroxyverbindungen enthaltender Rückstand zugegeben und das Sumpfprodukt in weitgehend flüssiger Form aus der Kolonne ausgetragen und anschließend einer Behandlung zur Rückgewinnung der Dihydroxyverbindung unterworfen wird.

## Claims

1. A process for working up residues which contain dihydroxy compounds and are obtained in the preparation of polyesters by reacting dicarboxylic acids or their esters or ester-forming derivatives with dihydroxy compounds,
a) in a first stage a diester of a dicarboxylic acid with an alkanol being subjected to transesterification with a molar excess of a dihydroxy compound,
b) the vapors which are obtained in the reaction according to a) and contain predominantly the alkanol formed in the transesterification, excess dihydroxy compounds and oligomeric and polymeric reaction products being transferred to a column in which the alkanol is separated off via the top and the other products are separated off as a bottom product, and
c) the bottom product being then subjected to a further treatment to recover the dihydroxy compound,
wherein a liquid residue containing dihydroxy compounds is added in stage b) of the process, and the bottom product is discharged in substantially liquid form from the column and then subjected to a treatment for recovering the dihydroxy compound.

## Revendications

1. Procédé pour le traitement de résidus contenant des composés dihydroxylés, qui sont formés lors de la préparation de polyesters par conversion d'acides dicarboxyliques, respectivement de leurs esters ou de dérivés formant des esters avec des composés dihydroxylés, où
a) on réestérifie, dans une première étape, un diester d'un acide dicarboxylique avec un alcanol avec un excès molaire d'un composé dihydroxylé,
b) les vapeurs produites lors de la conversion selon le point a), qui contiennent principalement l'alcanol formé lors de la réestérification, des composés dihydroxylés en excès, de même que des produits de réaction oligomères et polymères, sont transférées dans une colonne dans laquelle l'alcanol est récupéré en tête et les autres produits sont isolés sous forme de produits de bas de colonne, et
c) le produit de bas de colonne est ensuite soumis à un traitement supplémentaire pour la récupération du composé dihydroxylé,
caractérisé en ce qu'on ajoute, à l'étape b) du procédé, un résidu liquide contenant des composés dihydroxylés et en ce qu'on évacue le produit de bas de colonne sous forme essentiellement liquide de la colonne et en ce qu'on le soumet ensuite à un traitement en vue de la récupération du composé dihydroxylé.
